(19) Europäisches Patentamt / European Patent Office / Office européen des brevets

(11) **EP 2 931 376 B1**

(12) **EUROPEAN PATENT SPECIFICATION**

(45) Date of publication and mention
of the grant of the patent:
**26.07.2017 Bulletin 2017/30**

(51) Int Cl.:
*A61Q 5/00* *(2006.01)*          *A61Q 11/00* *(2006.01)*
*A61K 8/73* *(2006.01)*          *A61Q 19/00* *(2006.01)*

(21) Application number: **13792375.1**

(22) Date of filing: **18.11.2013**

(86) International application number:
**PCT/EP2013/074085**

(87) International publication number:
**WO 2014/095198 (26.06.2014 Gazette 2014/26)**

(54) **TOPICAL COMPOSITION**

TOPISCHE ZUSAMMENSETZUNG

COMPOSITION TOPIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2012 PCT/CN2012/086778**

(43) Date of publication of application:
**21.10.2015 Bulletin 2015/43**

(73) Proprietors:
• **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**
• **Unilever PLC**
**London, Greater London EC4Y 0DY (GB)**
Designated Contracting States:
**CY GB IE MT**

(72) Inventors:
• **BANERJEE, Gautam**
**Bangalore 560 066 (IN)**
• **MAJUMDAR, Amitabha**
**Bangalore 560 066 (IN)**
• **MATHAPATHI, Mruthyunjaya, Swamy**
**Bangalore 560 066 (IN)**
• **SAMPATH KUMAR, Ramya**
**Bangalore 560 066 (IN)**
• **WANG, Shunchun**
**Shanghai 201203 (CN)**

(74) Representative: **van den Brom, Coenraad Richard**
**Unilever Patent Group**
**Olivier van Noortlaan 120**
**3133 AT Vlaardingen (NL)**

(56) References cited:
EP-A1- 0 324 720          WO-A1-2011/069781
WO-A1-2014/012755          US-A1- 2011 097 356

• **SAPHALA DHITAL ET AL: "Purification of innate immunostimulant from green tea using a silkworm muscle contraction assay", DRUG DISCOVERIES & THERAPEUTICS, vol. 5, 1 January 2011 (2011-01-01), pages 18-25, XP055110260, ISSN: 1881-7831, DOI: 10.5582/ddt.v5.1.18**
• **DATABASE BIOSIS [Online] BIOSCIENCES INFORMATION SERVICE, PHILADELPHIA, PA, US; January 2009 (2009-01), LI YUAN-HONG ET AL: "Protective effect of green tea extracts on photoaging and photo-immunosuppression", XP002722396, Database accession no. PREV200900252995 & ZHONGHUA PIFUKE ZAZHI, vol. 42, no. 1, January 2009 (2009-01), pages 25-27, ISSN: 0412-4030, DOI: 10.3760/CMA.J.ISSN.0412-4030.2009.01.010**

Remarks:
The file contains technical information submitted after the application was filed and not included in this specification

**Description**

**Field of the invention**

The present invention relates to a composition suitable for topical application to the skin, oral mucosa and scalp. In particular, the invention relates to a composition having a polymer for enhancing immunity.

**Background of the invention**

People experience many stresses in their day-to-day life. Such stresses include mental pressures, environmental stresses caused by breathing polluted air, consuming contaminated water and food or exposure to ultra-violet and other radiations. Other sources of stress include exposure to continual changes in ambient temperature and other weather conditions. Continuous exposure of the body to external stresses are believed to cause reduction in the immunity of the human body to diseases, whether of microbiological causes or otherwise. There is continuous interest in improving the innate immune system. People generally do not prefer medicinal solutions to solving the problem of reduced immunity. This is because, although medicinal solutions are believed to be effective, they are believed to also cause undesirable side-effects. Thus, there is a continuing demand for "natural" solutions to enhancing the immunity.

A large number of herbs and natural materials have been part of traditional medicines and consumed by people over the centuries for various health benefits and enhancing innate immune system.

Extraction of polysaccharides from natural sources and their use in providing immunity benefits has been known.

WO2011069781A1 (Unilever) discloses a polysaccharide for modulating immune response, a method for isolation of the polysaccharide and edible products having the polysaccharide. This application discloses a polysaccharide obtained from plants of the species *Camellia sinensis* wherein the backbone of the polysaccharide comprises alternating rhamnogalacturonan-I cores and alpha(1,4)- linked polygalacturonic acid or alpha(1,4)-linked oligogalacturonic acid cores, wherein the molar ratio of galacturonyl acid residues to rhamnosyl residues in the backbone of the polysaccharide ranges from 2.5:1 to 1:1, and wherein the polysaccharide has a molecular weight of at least 70 kDa.

US2011097356 discloses an anti-inflammatory and antioxidant cosmetic which comprises green tea polysaccharide and Tricholoma matsutake extract. The green tea extract is prepared by hot water extraction followed by ethanol precipitation. There is no indication of any separation step, and no indication of the molecular weight of the product.

Drug Discoveries & Therapeutics, 2011, Vol.5(1), pp18-25 discloses innate immunostimulants based on green tea extracts. The extracts are prepared by extraction with hot water (using an autoclave) followed by ethanol. The extracts are comprised of polysaccharide based on galacturonic acid. The only information given in this document as to the molecular weight is that it seems to be between 500 kDa and 40 kDa (page 21, section 3.4). The galacturonic acid is 69% of the total monosaccharides, thus confirming that polygalacturonic acid is the major polysaccharide in green tea. The extracts are purified by several processes. At each stage, these polysaccharides are are dissolved in various solvents such as D2O, buffers, ethanol etc. It is clear that the fraction collected here has a molecular weight between 500 kDa and 40 kDa.

BIOSIS Abstract PREV200900252995 discloses the protective effect of green tea extracts on photoageing and photoimmunosuppression in the skin.

EP324720 discloses the use of polysaccharides such as polygalacturonic acid to inhibit plaque. No details are given of the molecular weight of the polygalacturonic acid.

US 2007/0202233 (Kato et al.) discloses a pectin having high molecular weight and a cosmetic material having the pectin for providing moist or smooth feeling. This application discloses high molecular weight pectin having 70 to 90 mole% galacturonic acid and use of the pectin in cosmetic material for preventing evaporation of moisture from skin, controlling moisture on the skin surface and for providing a moist feeling. The application further discloses the use of the pectin in face lotion, cleansing foam, soap, pressed powder, foundation, hair spray.

There is a desire to increase the natural defence of the human body against infections by using naturally occurring compounds or ingredients, especially as part of a common cosmetic or dermatological preparations, as the consumer generally does not want to rely on medicaments only.

It is thus an object of the present invention to provide a topical composition, especially oral, skin or hair composition which provides enhanced immunity benefits.

It is yet another object of the present invention to provide a process for producing an extract of *Camellia sinensis* which provides enhanced immunity benefits.

The present inventors have determined that a topical composition having a polymer of galacturonic acid with a molecular weight of 18kD to 35kD provides enhanced immunity benefits.

## Summary of the invention

According to a first aspect disclosed is a topical composition comprising:

(i) 0.1 to 10 wt% of a polymer of galacturonic acid; and,
(ii) a base;

characterised in that the polymer of galacturonic acid has a molecular weight from 18 kDa to 35 kDa, wherein the polymer of galacturonic acid is obtainable by a process comprising the steps of:

(i) extracting *Camellia sinensis* in water at a temperature of 85 to 100 °C;
(ii) separating the extracted Camellia *sinensis* to obtain an aqueous extract;
(iii) adding 30 to 70% ethanol-water solution to the aqueous extract to generate a precipitate;
(iv) separating the precipitate of step (iii);
(v) washing the precipitate of step (iv) with 70 to 95% ethanol-water solution;
(vi) filtering and drying the washed precipitate of step (v) to obtain the extract of *Camellia sinensis* comprising a polymer of galacturonic acid with a molecular weight from 18 to 35kDa.
(vii) separating the polymer of galacturonic acid with a molecular weight from 18 kD to 35 kDa from said extract.

According to a further aspect disclosed is a composition for use in a method of improving the immunity of skin, oral mucosa or scalp by applying to the desired surface a composition of the first aspect.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different end points are also contemplated.

## Detailed description of the invention

According to a first aspect there is provided a topical composition having a polymer of galacturonic acid and a base.

Polymer of galacturonic acid:

Disclosed composition includes 0.1 to 10 wt% of a polymer of galacturonic acid. Preferably the composition includes not less than 0.5 wt%, more preferably not less than 0.8 wt%, still preferably not less than 1 wt% and most preferably not less than 2wt% of the polymer of galacturonic acid. The composition preferably includes not more than 8 wt%, more preferably not more than 6 wt% still preferably not more than 5 wt%, further preferably not more than 4 wt% and most preferably not more than 3 wt% of the polymer of galacturonic acid.

The polymer of galacturonic acid has a molecular weight from 18 kDa to 35 kDa more preferably from 20 kD to 30 kD and still preferably 22 kD to 25 kD.

It is highly preferred that the polymer of galacturonic acid is free of side chains. Side chains, if present, is present on not more than 20%, preferably not more than 10%, further more preferably not more than 5% and even further more preferably not more than 2% of the galacturonic acid group present on the polymer chain. It is further preferred that the polymer is a homopolymer of galacturonic acid.

Preferably the source of the polymer of galacturonic acid is *Camellia sinensis*. Preferably the polymer of galacturonic acid is from *Camellia sinensis var. sinensis, Camellia sinensis var. assamica* or *Camellia sinensis* var *japonica.* There is no particular preference for the part of the plant that has to be taken for the extraction purpose. Leaf, buds stem or any other part of the *Camellia sinensis* may be used for the purpose of the extraction. Even a mixture of different parts of the Camellia *sinensis* may be used.

When the source of the polymer of galacturonic acid is *Camellia sinensis* the composition of the present invention preferably includes 1 to 20 wt% of an extract of *Camellia sinensis*. It is preferred that the composition includes not more than 18 wt%, further preferably not more than 16 wt% still further preferably not more than 12 wt% of the extract of *Camellia sinensis.* It is preferred that the composition includes not less than 2 wt% , further preferably not less than 5 wt% and further preferably not less than 8 wt% of the extract of *Camellia sinensis.*

It is preferred that the *Camellia sinensis* is green tea leaves.

"Green tea" refers to substantially unfermented tea. "Fermentation" refers to the oxidative and hydrolytic process that tea undergoes when certain endogenous enzymes and substrates are brought together, e.g., by mechanical disruption of the cells by maceration of the leaves. Green tea is generally prepared by heat treating freshly picked leaves to arrest enzyme action and then subjecting the leaves to a series of drying and rolling steps.

The preparation of the green tea leaves preferably includes the following steps:

*Providing fresh leaves:* In its simplest form, the fresh tea leaves are provided in freshly plucked form, i.e. without any further processing, and have a moisture content of 76 to 80 wt%. The fresh tea leaves preferably comprise leaf and stem material. Most preferably the fresh tea leaves comprise actively growing buds, e.g. in the form of the first two or three leaves together with the unopened bud (so-called "two-and-a-bud" and/or "three-and-a-bud" material).

*Heat treatment:* The fresh tea leaves are then preferably subjected to heat treatment. The heat-treatment of the fresh leaves arrests the enzyme action. Heat treatment must preferably be sufficient to inactivate endogenous enzymes responsible for fermentation during or after maceration. It is preferred that the moisture content of the leaves is controlled in the range of 74 to 76 wt%. Suitable heat-treatments are well-known to those skilled in the art and include steaming and/or pan-firing (see, for example, " Tea: Cultivation to Consumption", K.C. Willson and M.N. Clifford (Eds), 1st Edn, 1992, Chapman and Hall (London), Chapter 13). Steaming is the preferred mode of heat treatment as this avoids scorching of the leaf surface which is sometimes encountered in contact-heating such as pan-firing. As the fermentation is arrested by the inactivation of enzyme polyphenol oxidase, the polyphenols are not oxidized and the leaves remain green.

*Maceration:* Following the heat treatment the fresh leaves are subjected to maceration. Maceration serves two functions. Firstly it damages the leaves such that their contents are more or less accessible to water used to make a beverage. Secondly it changes the shape and size of the leaves. Traditionally, green tea is macerated using batch equipment known as rollers (see, for example, " Tea: Cultivation to Consumption", K.C. Willson and M.N. Clifford (Eds), 1st Edn, 1992, Chapman and Hall (London), Chapter 13) or by rolling by hand. Green teas are also known that are macerated using continuous equipment such as CTC machines or using a combination of rotorvane and double-cone processor. The preferred arrangement is one in which the leaves are first passed through the rotorvane to produce partially macerated leaves and then passed through the double-cone processor to produce the macerated leaves. The maceration is particularly effective if the maceration is performed on fresh leaves having not too high a moisture content. Thus it is preferred that the fresh leaves are partially dried to a moisture content of from 65 to 70 wt% prior to maceration. Maceration is also most effective if the leaves are at a low temperature. Thus it is preferred that the fresh leaves are cooled to a temperature of from 5 to 40°C prior to maceration.

*Drying:* Following maceration the leaves are dried. To allow for long-term storage stability, it is preferred that the leaves are dried to a moisture content of less than 30 wt%, more preferably from 1 to 10 wt% and most preferably 3 to 4 wt% of the leaves. Suitable drying processes are known in the art and include tray drying. However, it is highly preferred that the drying step comprises drying the macerated leaves in a fluid bed dryer as this allows for more uniform heating.

*Sorting:* The dried macerated leaves are preferably sorted according to particle size. In particular it is preferred that the leaves are sorted to recover those particles with a size larger than 30 mesh (595 micrometres), more preferably above 25 mesh (707 micrometres) and most preferably above 20 mesh (841 micrometres). Sorting may also involve recovering those particles with a particle size below 3 mesh (5.66 mm), more preferably below 4 mesh (4.76 mm) and most preferably below 5 mesh (4.00 mm). Suitably the step of sorting the leaves comprises sieving the dried macerated leaves.

Base

Disclosed composition may be a skin composition or a hair composition or an oral composition. The composition according to the invention includes a base. It is preferred that depending on whether the topical composition is a skin, hair or oral composition the base is respectively a dematologically/cosmetically acceptable base or an orally acceptable base. The base preferably acts as a diluant, dispersant or carrier for the polymer of galacturonic acid and facilitates its distribution when the composition is applied to the desired surface.

Skin composition

"Skin Composition" as used herein, is meant to include a composition for topical application to skin of mammals, especially humans. Such a composition may be generally classified as leave-on or rinse off, and includes any product applied to a human body for improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of skin compositions include leave-on skin lotions and creams, shampoos, conditioners, shower gels, toilet bars, antiperspirants, deodorants, depilatories, lipsticks, foundations, mascara, sunless tanners and sunscreen lotions. "Skin" as used herein is meant to include skin on the face and body (e.g., neck, chest, back, arms, underarms, hands, legs and buttocks).

When the topical composition is a skin composition it includes a base preferably a dermatologically/cosmetically acceptable base. The base acts as a diluant, dispersant or carrier for the polymer of galacturonic acid according to the present invention. The base may include materials commonly employed in skin compositions such as water, liquid or solid emollients, propellants, powders, emulsifiers, solvents, humectants, thickeners,. The base in the skin composition may be a single or mixtures of one or more vehicles.

Suitable emollients for the skin composition includes but is not limited to stearyl alcohol, glycerol monoricinoleate, glycerol monostearate, mink oil, cetyl alcohol, isopropyl isostearate, stearic acid, isobutyl palmitate, isocetyl stearate, oleyl alcohol, isopropyl luarate, hexyl laurate, decyl oleate, octadecan-2-ol, isocetyl alcohol, eicosanylalcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernal oil, rapeseed oil, safflower seed oil, evening primrose oil, soybean oil, sunflower seed oil, avocado oil, olive oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate or myristyl myristate.

Suitable propellants for the skin composition includes but is not limited to trichlorofluoromethane, dichlorodifluoro- methane, dichlorotetrafluoroethane, monochlorodifluoromethane, trichlorot fluoroethane, propane, butane isobutanem demethyl ether, carbon dioxide, nitrous oxide; solvents such as ethyl alcohol, methylene chloride, isopropanol, acetone, ethylene glycol monoethyl ether, diethlyene glycol monobutyl ether, diethylene glycol monoethyl ether, dimethyl sulphoxide, dimethyl formamide, tetrahydrofuran.

Suitable powders for the skin composition includes but is not limited to chalk, talc, fullers earth, kaolin, starch, gums, colloidal silica sodium polacrylate, tetre alkyl and/or trialkyl aryl ammonium smectites, chemically modified magnesium aluminium silicate, organically modified montmorillonite clay, hydrated aluminium silicate, fumed silica, carboxyvinyl polmer, sodium carboxymethyl cellulose, ethylene glycol monostearate.

Preferably the base is present in the skin composition from 80 wt% to 99 wt%, preferably from 85 wt% to 90 wt%.

The skin composition according to the present invention may be prepared by the usual manner for preparing skin care products. The polymer of galacturonic acid is generally incorporated in the dermatologically or cosmetically acceptable base in a conventional manner. The polymer of galacturonic acid can suitably first be dissolved or dispersed in a portion of the water or another solvent or liquid to be incorporated in the composition. The preferred skin compositions may be an oil-in-water or water-in-oil emulsions.

The skin composition may be in the form of conventional skin-care products such as a cream, gel or lotion or the like. The composition can also be in the form of a so-called "wash-off" product example; a bath or shower gel, possibly containing a delivery system for the polymer of galacturonic acid to promote adherence to the skin during rinsing. Most preferably the skin composition is a "leave-on" product; a product to be applied to the skin without a deliberate rinsing step soon after its application to the skin.

Cream:

A preferred skin composition according to the present invention includes a cream base having 1 to 25 wt% fatty acid and preferably having 0.1 to 10 wt% soap. A still preferred cream base is a vanishing cream. Vanishing cream preferably includes 5 to 25 wt% fatty acid and 0.1 to 10 wt% soap. $C_{12}$ to $C_{20}$ fatty acids are especially preferred in vanishing cream bases, further more preferred being $C_{14}$ to $C_{18}$ fatty acids. The most preferred fatty acid is stearic acid. The fatty acid may also be a mixture of palmitic and stearic acid. The fatty acid in the composition is more preferably present in an amount in the range of 5 to 20 wt% of the composition.

Soaps in the vanishing cream base preferably include alkali metal salt of fatty acids, like sodium or potassium salts, most preferred being potassium stearate. The soap in the vanishing cream base is generally present in an amount in the range of 0.1 to 10 wt%, more preferably 0.1 to 3 wt% of the composition. Generally the vanishing cream base in cosmetic compositions is prepared by taking a desired amount of total fatty matter and mixing with potassium hydroxide in desired amounts. The soap is usually formed in-situ during the mixing. The skin composition when formulated as a vanishing cream preferably includes 60 to 85 wt%, more preferably 65 to 80 wt% water.

Deodorant product:

Disclosed skin composition may be a deodorant product that can generally be in the form of firm solids, soft solids, gels, creams, and liquids and are dispensed using applicators appropriate to the physical characteristics of the composition. Deodorant product may also be in the form of an emulsion or a microemulsion.

Deodorant compositions which are in liquid form generally include a liquid carrier. Such liquid carrier can be hydrophobic or has a mixture of both hydrophilic and hydrophobic liquids. The liquid carrier or mixture of carriers often constitutes from 30 to 95 wt% and in many instances from 40 to 80 wt% of the composition. Hydrophobic liquid carriers commonly can include one or more materials selected within the chemical classes of siloxanes, hydrocarbons, branched aliphatic alcohols, esters and ethers that have a melting point not higher than 25°C and a boiling point of at least 100°C. Hydrophilic carrier liquids that can be employed in compositions herein commonly include water and/or a mono or polyhydric alcohol or water-miscible homologue. Monohydric alcohols often are short chain, by which is meant that they contain up to 6 carbons, and in practice is most often ethanol or sometimes iso-propanol. Polyhydric alcohols commonly include ethylene or propylene glycol, or a homologue can be employed such as diethylene glycol. The deodorant compositions that remain in liquid form can be applied employing conventional applicators such as a roll-on or by being pumped or squeezed through a spray-generating orifice. Such compositions may be thickened, for example using one or more thickeners described subsequently herein.

Compositions that are firm solids, commonly obtained by use of a gellant or structurant, can be applied employing a stick applicator and soft solids, gels and creams can be applied employing an applicator having a dispensing head provided with at least one aperture through which the soft solid, gel or cream can be extruded under mild pressure. Suitable thickeners or gellants that may be used for achieving this is by use of water-soluble or dispersible materials of higher viscosity, including various of the emulsifiers, and/or thickened or gelled with water-soluble or water-dispersible polymers including polyacrylates, and water-soluble or dispersible natural polymers, such as water-soluble polysaccharide or starch derivatives, such as alginates, carageenan, agarose and water-dispersible polymers include cellulose derivatives. The concentration of such polymers in the water-immiscible liquid is often selected in the range of from 1 to 20 wt% depending on the extent of thickening or structuring required, and the effectiveness of the chosen polymer in the liquid/mixture.

Suitable structurants that are desirable by virtue of its long standing proven capability to produce firm solids and more recently in making soft solids includes waxes. Herein, the term wax is employed to encompass not only materials of natural origin that are solid with a waxy feel and water-insoluble at 30-40°C, but melt at a somewhat higher temperature, typically between 50 and 95°C, such as beeswax, candelilla or carnauba wax, but also materials having similar properties. Such other waxes include hydrocarbon waxes, example paraffin wax, mineral wax and microcrystalline wax; synthetic waxes, such as polyethylene of 2000 to 10000 daltons; waxy derivatives or waxy components of natural waxes. Mixtures of materials within each class of gellant/ structurant can be employed.

When a deodorant composition employed herein comprises an aerosol composition, it contains a propellant in addition to a base composition as described herein above, commonly in a weight ratio of from 95:5 to 40:60, and in many formulations, the weight ratio is from 90:10 to 50:50. The propellant is conveniently a low boiling point material, typically boiling below -5°C, for example an alkane such as propane, butane or isobutane, and possibly containing a fraction of pentane or isopentane, or a hydrofluorocarbon or fluorocarbon of similar carbon content. During filling of the aerosol canister, the propellant gas is liquified by virtue of the elevated pressure that is generated therein.

Optional skin care ingredients:

Skin compositions of the present invention can include a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The skin composition is preferably intended to be a product for topical application to human skin, especially as an agent to improve the immunity of the skin.

In use, a quantity of the composition, for example from 1 to 100 ml, is applied to exposed areas of the skin, from a suitable container or applicator and, if necessary, it is then spread over and/or rubbed into the skin using the hand or fingers or a suitable device.

The composition may be packaged in any suitable container such as in a jar, a bottle, tube, roll-ball, or the like, in the conventional manner to suit its viscosity and intended use by the consumer. For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lid jar.

Hair Composition

When the topical composition according to the present invention is a hair composition it includes a base preferably a dematologically/cosmetically acceptable base. The hair composition may suitably take the form of shampoos, conditioners, sprays, mousses, tonics, gels, oils, creams, air infused styling foams, rinses or lotions.

The choice of appropriate base will depend on the form of the hair composition and also depend on whether the product formulated is meant to be left on the surface to which it is applied (e.g., hair spray, mousse, tonic, or gel), or rinsed off after use (e.g., shampoo, conditioner, rinses).

The base used in the disclosed hair composition can be in a wide variety of forms. For example it may be an oil-in-water, water-in-oil, water-in-oil-in-water, and oil-in-water-in-silicone emulsions. The dermatologically/cosmetically acceptable base used herein can include a wide range of components conventionally used in hair compositions. The base can be a solvent to dissolve or disperse the polymer of galacturonic acid and is preferably the $C_1$-$C_6$ alcohols, lower alkyl acetate and mixtures thereof. The base can also contain a wide variety of additional materials such as acetone, hydrocarbons (such as isobutane, hexane, decene), halogenated hydrocarbons (such as Freons) and volatile silicon derivatives such as cyclomethicone.

When the hair care composition is a hair spray, tonic, gel, air infused styling foams or mousse the preferred solvent base include water, ethanol, volatile silicone derivatives, and mixtures thereof. The solvents used in such mixtures may be miscible or immiscible with each other. Other suitable base preferably includes anhydrous liquid solvents such as oils, alcohols, and silicones (e.g., mineral oil, ethanol, isopropanol, dimethicone, cyclomethicone, and the like); aqueous-based single phase liquid solvents (e.g., hydro-alcoholic solvent systems); and thickened versions of these anhydrous and aqueous-based single phase solvents (e.g., where the viscosity of the solvent has been increased to form a solid or semi-solid preferably by the addition of appropriate gums, resins, waxes, polymers, salts, and the like). Mousses and aerosol hair sprays can also utilise any of the conventional propellants to deliver the material as a foam (in the case of a mousse) or as a fine, uniform spray (in the case of an aerosol hair spray). Examples of suitable propellants preferably include materials such as trichlorofluoromethane, dichlorodifluoromethane, difluoroethane, dimethylether, propane, n-butane or isobutane. The level of propellant can be preferably adjusted as desired but is generally from about 3 % to about 30 % of mousse compositions and from about 15 % to about 50 % of the aerosol hair spray compositions. A tonic or hair spray product having a low viscosity may also utilise an emulsifying agent. Examples of suitable emulsifying agents preferably include nonionic, cationic, anionic surfactants, or mixtures thereof.

Suitable spray containers are well known in the art and preferably include conventional, non-aerosol pump sprays i.e., "atomisers", aerosol containers or cans having propellant, as described above, and also pump aerosol containers utilising compressed air as the propellant. Where the hair composition are conditioners and rinses the carrier can preferably include a wide variety of conditioning materials. Where the hair compositions are shampoos, the base can preferably include, for example, surfactants, suspending agents, and thickeners.

Depending on the type of composition employed, one or more additional ingredients conventionally incorporated into hair treatment compositions may be preferably included in compositions of the invention. Such additional ingredients include styling agents, such as resins and hair-setting polymers, perfumes, dyes, buffering or pH adjusting agents, viscosity modifiers, opacifiers, pearlescers, preservatives, antibacterial agents, antidandruff agents, foam boosters, proteins, moisturising agents, herb or other plant extracts and other natural ingredients.

Oral composition

When the topical composition according to the present invention is an oral composition it includes a base, preferably an orally acceptable base. The orally acceptable base depends on the format in which the oral care composition is delivered. Most suitable formats are mouthwash, a toothpaste or a toothpowder.

Mouthwash:

When the composition is formulated as a mouthwash, the orally acceptable base is water.

The mouthwash of the invention preferably includes 0.05 to 10 wt%, more preferably 0.05 to 8 wt%, most preferably 0.5 to 5 wt% of a surfactant. The surfactant is preferably cationic, anionic, or zwitterionic, most preferably cationic. When an anionic surfactant is present it is preferably chosen from alkali or alkaline earth metal salts of alkyl sulphonic acid, fatty acid, or alkyl ether sulphate. When a zwitterionic surfactant is present it is preferably chosen from betaines, sulphobetains, hydroxyl sulphobetains, or amino carboxylates. When a cationic surfactant is present it is preferably benzalkonium chloride, alkyl pyridinium chloride or quaternary ammonium gemini surfactants. The mouthwash composition of the invention is used for improving immunity either by using the composition with no dilution or after diluting the composition with water. The preferred weight ratio of composition to water for the dilution step is in the range of 1:1 to 1:200, more preferably 1:5 to 1:50, further more preferably 1:15 to 1:30 and ideally about 1:20.

Toothpaste:

The oral care composition may be delivered in a toothpaste format. When the composition is a toothpaste, the orally acceptable base is an abrasive which may be calcium carbonate or abrasive silica. When calcium carbonate is the abrasive, the toothpaste is in an opaque paste format. When abrasive silica is used, the toothpaste is usually delivered in a transparent gel format. The toothpaste preferably includes 2 to 15 wt% of a surfactant, preferably 2.2 to 10 wt%, more preferably 2.5 to 5 wt% of the total composition. Preferred surfactants are anionic or amphoteric in nature. The anionic surfactant is preferably an alkali metal alkyl sulphate, more preferably a sodium lauryl sulphate (SLS). Mixtures of anionic surfactants may also be employed. The amphoteric surfactant is preferably a betaine, more preferably an alkylamidopropyl betaine (wherein the alkyl group is a linear $C_{10}$~$C_{18}$ chain), and most preferably is cocoamidopropyl betaine (CAPB). Mixtures of amphoteric surfactants may also be employed.

Opaque toothpaste:

Preferably the orally acceptable base in opaque toothpaste includes 15 to 70 wt% calcium carbonate, more preferably 30 to 60 wt%. Calcium carbonate (also known as chalk) is available in many forms and some of these forms are used in oral care compositions. Two commonly used forms are FGNC (fine ground natural chalk) and PCC (precipitated calcium carbonate). Of the total chalk content in the oral care composition, FGNC, is generally present in 35 to 100% preferably from 75 to 100% and especially from 95 to 100%, the balance being PCC. Typically, the compositions include 30 to 65 wt%, preferably 35 to 55 wt% and most preferably from 40 to 55 wt% of FGNC. FGNC generally includes particles of weight-based median particle size preferably of 1 to 15 $\mu$m, more preferably of 2 to 10 $\mu$m and further preferably of 4 to 7 $\mu$m. The composition may also include other known "non-chalk" abrasives to improve the abrasive action. Such abrasives include dicalcium phosphate dihydrate (DCPD) and silica. In addition to calcium carbonate, one can also include abrasive silica in opaque toothpastes for enhanced abrasive action. When present, the composition includes 4 to 15 wt%, preferably 6 to 12 wt%, and further more preferably 7 to 10 wt% of abrasive silica. Alternatively the composition may include 0.0.1 to 2 wt%, preferably 0.1 to 0.8 wt% and more preferably 0.3 to 0.7 wt% of perlite. Water in the opaque toothpaste is generally included at 15 to 40 wt%, preferably 20 to 30 wt% of the composition. Preferred compositions include a humectant, e.g. xylitol, glycerol or sorbitol. Glycerol and sorbitol are particularly preferred. Preferably, the compositions include 0.1 to 20 wt% humectants, more preferably 1 to 15 wt%, most preferably 5 to 13 wt%. Preferably the composition also includes an alkali-metal bicarbonate salt. Preferably the alkali-metal bicarbonate salt is a sodium salt. The composition preferably includes 1 to 30 wt%, more preferably 2 to 20 wt% and optimally 3 to 8 wt% of the alkali-metal bicarbonate salt.

*Gel toothpaste:*

Gel toothpaste preferably includes abrasive silica. The preferred abrasive silica has a low refractive index of 1.41 to 1.47 and more preferably of 1.435 to 1.445. Preferably the abrasive silica has a weight mean particle size of between 5 and 15 micrometers, a BET (nitrogen) surface area of between 10 and 100 m2/g and an oil absorption of about 70 - 150 cm3/100 g. Typical examples of suitable low refractive index abrasive silicas are Tixosil 63 and 73 (ex Rhone Poulenc); Sident 10 (ex Degussa); Zeodent 113 (ex Zeofinn); Zeodent 124 (ex Huber), Sorbosil AC series supplied by Crosfield such as Sorbosil ACII, Sorbosil AC39 and Sorbosil AC35, particularly Sorbosil AC 77 (ex Crosfield Chemicals). The amount of silica in the gel toothpaste composition is preferably 2 to 60 wt%, usually 2 to 20 wt% and more preferably 5 to 12 wt%. Thickening silica may also be incorporated in gel toothpaste, usually at 4 to 12 %, preferably 5 to 10% by weight of the composition. Examples of preferred grades of medium thickening silica includes MFIL (ex. Madhu Silica India), TC15 (from PQ Corp UK), Zeodent 165 (ex. Huber), or Tixosil 43 (from Rhodia). Water in the gel toothpaste is generally included at 8 to 14%, preferably 8 to 10% by weight of the composition. These amounts of water are exclusive of water which are incorporated in the composition from aqueous solutions of other ingredients e.g. sorbitol.The opaque or gel type of toothpaste may also include an anti-caries agent, binders, thickeners, flavours, stabilizing agents, polymers, vitamins, buffers and anti-calculus agents.

Toothpowder:

Toothpowders usually have very high percentage of abrasives. Chalk (FGNC) is the most preferred abrasive but PCC may also be used. The desired amount of foam is provided by including an anionic surfactant e.g. Sodium Lauryl Sulphate in the toothpowder composition. Preferably the composition includes 2 to 3 wt% of surfactant. Other ingredients like silica or sodium monofluorophosphate may be included at up to 1 wt% in the composition. Sweeteners such as xylitol, sorbitol, glycerol or sachharin may be included. Flavours such as spearmint or peppermint may be preferably included upto 1 wt% of the toothpowder composition.

Thus, in the disclosed composition the orally acceptable base is preferably selected from water, silica or calcium carbonate.

Preferably the topical composition according to the present invention is a leave-on or a rinse-off product.

Process of obtaining polymer of galacturonic acid:

According to a second aspect disclosed polymer of galacturonic acid is obtainable by a process comprising the steps of: (i) extracting *Camellia sinensis in* water at a temperature of 85 to 100°C; (ii) separating the extracted *Camellia sinensis* to obtain an aqueous extract; (iii) adding 30 to 70 % ethanol-water solution to the aqueous extract of to generate a precipitate; (iv) separating the precipitate of step (iii); (v) washing the precipitate of step (iv) with 70 to 95 % ethanol-water solution; (vi) filtering and drying the washed precipitate of step (v) to obtain the extract of *Camellia sinensis* comprising a polymer of of galacturonic acid with a molecular weight from 18 to 35kDa; (vii) separating the polymer of galacturonic acid with a molecular weight from 18 to 35kDa from said extract of *Camellia sinensis.*

The step of extracting *Camellia sinensis* includes extracting in water at a temperature of 85 to 100°C, preferably at a temperature in the range of 90 to 100°C and more preferably in between 95 to 100°C. Preferably the weight ratio of plant part of *Camellia sinensis* to water is 1:1 to 1:20 more preferably 1:1 to 1:15 and most preferably from 1:1 to 1:10. The extraction in hot water at a temperature of 85 to 100°C is preferably conducted for a period of about 60 to 180 minutes more preferably from 80 to 180 minutes and further preferably from 100 to 180 minutes and most preferably from 120 to 180 minutes.

The extraction step is followed by separating the extracted *Camellia sinensis* to obtain an aqueous extract. The separation is preferably by filtration.

The separation step is followed by addition of 30 to 70% ethanol-water solution to the aqueous extract to form a precipitate. Preferably 35 to 60% and more preferably 40 to 50% of an ethanol-water solution is used.

The precipitate is separated preferably by centrifugation thereby producing a sediment and a supernatant. After the centrifugation the sediment is washed with 70 to 95 % ethanol-water solution, preferably with 75 to 90 % and more preferably with 85 to 90 % ethanol-water solution. The supernatant produced may further preferably be treated by adding 60 to 85% ethanol-water solution, more preferably with 65 to 85% and still preferably with 70 to 80% ethanol-water solution followed by centrifugation to produce further sediment. Preferably the sediment is then further washed with 30 to 50 % ethanol, preferably with 35 to 45% of ethanol.

After the washing step the precipitate is filtered and dried to obtain the extract of *Camellia sinensis* comprising a polymer of galacturonic acid with a molecular weight from 18 to 35kDa. Preferably the drying step is carried out in a vacuum dryer.

The extract of *Camellia sinensis* comprising a polymer of galacturonic acid with a molecular weight from 18 to 35kDa is preferably subjected to a separation step comprising the separation of the fraction of polymer of galacturonic acid with a molecular weight from 18 to 35kDa. Separation is preferably by dialysis, size exclusion chromatography.

According to a third aspect disclosed is a method of improving the immunity of skin, oral mucosa or scalp by applying to the desired surface a composition of the first aspect.

The method according to the present invention preferably is a non-therapeutic method of improving immunity. Preferably the method of the present invention may be carried out one or more times daily by applying to the skin, oral mucosa or scalp which requires improvement in immunity. The amount of the composition used and the frequency with which it is applied may vary. In general, a small quantity of the composition, for example from 0.1 to 5 ml is applied to the skin, scalp or oral mucosa. A rinsing step may optionally follow depending on whether the composition is formulated as a "leave-on" or a "rinse-off" product.

According to a fourth aspect disclosed is use of a composition according to the first aspect for enhancing immunity of the skin, hair or scalp. The use of the composition according to the present invention preferably is a non-therapeutic method of improving immunity.

Now the invention will be demonstrated by means of following non-limiting examples.

The examples given below are only for illustrations and in no way limits the scope of the invention.

**Examples**

Preparation of polymer of galacturonic acid from an extract of Camellia *sinensis*:

For this experiment commercial samples of Wufeng green tea leaves were purchased from market in city of Wuhan, Hubei province in China.

A batch of 2.5 kg of the green tea leaves was suspended in 25 liters of water and extracted at a temperature of 100°C for 2 hours. The tea leaves after extraction were removed by filtration and the aqueous extract was collected. The extracted tea leaves were further extracted under the same conditions and then filtered to obtain a second batch of aqueous extract. The aqueous extract obtained in the first extraction step and the second extraction steps were combined and concentrated.

To the concentrated aqueous extract, ethanol was added till the concentration of ethanol reached 70% concentration to precipitate out the polysaccharide. The resulting precipitate was centrifugation at ~9829g for about 10 minutes, collected and was then washed twice with 95% ethanol followed by freeze drying to obtain the polysaccharide extract of *Camellia sinensis.*

5 grams of the polysaccharide extract of *Camellia sinensis* prepared by extraction process described above was dissolved in 40 mL distilled water and centrifuged (43540xg, 10 minutes). The supernatant obtained after centrifugation was collected and the sediment was dissolved in 20 mL distilled water and again centrifuged (43540xg, 10 minutes) to remove the residue. Supernatant obtained from the first and the second centrifugation were combined and loaded on DEAE-Sepharose Fast Flow column (45x5 cm) previously treated with 2.0 M NaCl and equilibrated with distilled water. The polysaccharide in the tea extract were first eluted with distilled water and then with increasing molarity (0.1 M, 0.2 M, 0.4 M and 2.0 M) of NaCl, sequentially. The sugar content of each fraction was assayed by phenol sulphuric acid method using galactose as a standard material, to obtain an elution curve. Each fraction was collected, concentrated, dialyzed with 3000 cut-off dialysis bag and freeze dried. The weight of the fraction eluted with 0.2M of NaCl constituted approximately 200mg.

The fraction obtained with 0.2M of NaCl was further purified by size exclusion chromatography on a Sephacryl S-300 high resolution column (60x2.6 cm) to obtain a purified sample of the polymer of galacturonic acid.

Homogeneity and molecular weight was determined by high performance gel permeation chromatography (HPGPC, KS-804 and KS-805 column in serials, ID 8 mm, and length 300 mm, Shodex) and the molecular weight of the polymer of galacturonic acid was found to be 18 kD to 35 kD.

Example 1: Evaluation of psoriasin gene expression in the untreated keratinocyte cell and the keratinocyte cell treated with the polymer of galacturonic acid according to the present invention.

Evaluation of the ability of polymer of galacturonic acid to enhance the immunity is done by measuring levels of expression of psoriasin gene in keratinocyte cells. Increase in the activity of the psoriasin genes in the keratinocyte cells is associated with improvement in the immunity response of the cells.

*Step 1: Preparation of lysate of primary keratinocyte cells:*

Materials

**[0081]**

　　1) 5mg/mL stock solution of polymer of galacturonic acid: 5milligrams of the polymer of galacturonic acid was dissolved in 1 mL of keratinocyte growth medium.
　　2) Primary keratinocyte culture (extracted from human skin)
　　3) Keratinocyte growth medium (KGM, from Epilife media).
　　4) Lysis buffer: Lysis buffer was prepared by addition of 10µg/mL of • -mercapto ethanol to RLT buffer (procured from Qiagen, Hilden, Germany).

Method

A 6 well plate was taken. 70,000 primary keratinocyte cells per well were taken and cultured in 1 mL of the KGM (Epilife media) to 70 to 80% confluency.

2 wells were marked as Control (C). The 2 control wells had a mixture of the primary keratinocyte cells and the KGM media. The Control keratinocyte cells were not treated with the polymer of galacturonic acid.

2 other wells were marked as Ex 1. Wells marked Ex 1 had a mixture of 1 mL of primary keratinocyte cells and the KGM media. The keratinocyte cells in wells marked Ex 1 were treated with 100μg of the polymer of galacturonic acid.

The remaining 2 wells were marked as Ex 2. Wells marked Ex 2 had a mixture of 1 mL of primary keratinocyte cells and the KGM media. The keratinocyte cells were treated with 500μg of the polymer of galacturonic acid.

The cells in the 6 wells were incubated at 37°C for 16 to 18 hours. After the incubation period the spent media from the control well (C), the 2 wells treated with 100μg/ml of the polymer of galacturonic acid (Ex 1) and the 2 wells treated with 500μg/ml of the polymer of galacturonic acid (Ex 2) were stored at -70°C. The spent media was later used for the TNF• by ELISA method.

250μL of the lysis buffer was added to the cells in each of the wells and after 2 to 3 minutes the cells were flushed 8 to 10 times with a pipette to obtain a lysate. The lysate from each of the wells was stored separately at -70°C.

*Step 2: Isolation of RNA:*

Materials

1) Qiagen- RNeasy Mini Kit (Catalog No. 74106).
2) Primary keratinocyte lysate treated with 70% ethanol
3) DNAase I stock from Qiagen

Method:

Stored primary keratinocyte cell lysate obtained from the control wells was taken and treated with 70% ethanol. Similarly the primary keratinocyte cells from the wells marked Ex1 and Ex 2 were also treated with 70% ethanol. After the treatment RNA was extracted from the lysates using the Qiagen- RNeasy™ minikit (catalog No. 74106) according to the manufacturers' instructions and the purified RNA was collected from the control, Ex 1 and Ex 2 respectively.

*Step 3: Synthesis of cDNA*

Materials

1) 5x RT buffer (from Bio-Rad Laboratories, Inc.)
2) Reverse transcriptase (i Script™ cDNA synthesis Kit from Bio-Rad Laboratories, Inc.)
3) Purified RNA (obtained from the control, Ex1, Ex 2 keratinocyte cell lysate as described in Step 2)
4) Water

Method

To a PCR tube a reaction mixture of 2.0μL of 5x RT buffer, 0.5μL of reverse transcriptase, 7.5μL of Control RNA sample (500ng) and water was added. The PCR tube was placed in a Thermal cycler (from Bio-Rad Laboratories, Inc.) and incubated at 65°C for 5 minutes for pre-denaturation of Control RNA sample. The reaction mixture was then snap-chilled on ice for 1 minute and 2.5 μL of a mixture of 5x RT buffer (2.0μL) and reverse transcriptase (0.5μL) was added. The reaction mixture was again placed in the Thermal cycler and incubated at 25°C for 5 minutes followed by incubation at 42°C for 30 minutes for the synthesis of cDNA and thereafter incubated at 85°C for 5 minutes for final denaturation. The cDNA was incubated at 4°C for further use in PCR.

The above procedure for the synthesis of cDNA was similarly conducted for the RNA obtained from the Ex1 and Ex 2 keratinocyte cell lysates.

Step 4: *Real time PCR by SYBR Green Method (Bio-Rad)*

Materials

1) 2x PCR SYBR green buffer (Bio-Rad Laboratories, Inc.)
2) Primers (forward) 1.5pm (Bio-Rad Laboratories, Inc.)

3) Primers (reverse) 1.5pm (Bio-Rad Laboratories, Inc.)
4) Template (cDNA) 50ng (Control cDNA, Ex 1 cDNA, Ex 2 cDNA obtained by synthesis of cDNA as described in Step 3)
5) Water

Method

To a PCR tube a reaction mixture of 12.5 µl of 2x PCR sybr green buffer, 1.0 µl of Primers (forward) 1.5pm, 1.0 µl of Primers (reverse) 1.5pm, 5.0 µl of the Control cDNA (Template, 50ng) and 5.5 µl water was added. The working concentration of the template (cDNA) in the reaction mixture was 1.5pm.

The PCR tube was placed in a Thermal cycler (from Bio-Rad Laboratories, Inc.) set at a constant lid mode temperature of 100°C and an auto shut off set at a temperature below 30°C. The reaction mixture in the PCR tube was provided with the following incubation cycle:

1) Incubation at 95°C for a period of 5 minutes
2) Incubation at 95°C for a period of 15 seconds
3) Incubation at 60°C for a period of 30 seconds
4) Incubation at 72°C for a period of 30 seconds
5) Steps 2 to 4 were repeated for a further 49 times.

After the completion of the incubation cycle, the melting curve from 60 °C to 90 °C was read and the amount of psoriasin gene expressed was quantified and provided in Table 1.

Similarly the Ex1 and Ex 2 cDNA (template) were also subjected to Real time PCR as described for the control cDNA and after the completion of the incubation cycle the amount of psoriasin gene expressed for Ex 1 and Ex 2 were quantified and are provided in Table 1.

TABLE 1

| Psoriasin gene expression | |
|---|---|
| Examples | Fold change |
| Control (C) | 1.0 |
| Ex 1 (treated with 100µg polymer of galacturonic acid per mL media) | 2.2 |
| Ex 2 (500µg of polymer of galacturonic acid per mL media) | 2.6 |

The data in Table 1 clearly shows that the keratinocyte cells treated with the polymer of galacturonic acid according to the present invention (Ex 1, Ex 2) provides an increased expression of the psoriasin gene as compared to the untreated keratinocyte cells (control). The keratinocyte cells treated polymer of galacturonic acid (Ex 1, Ex 2) shows an improvement in the immune efficacy as compared to the untreated keratinocyte cells (Control).

Example 2: Evaluation of the supernatant media obtained from the keratinocyte cell treated with the polymer of galacturonic acid and the untreated keratinocyte cells by Enzyme linked immune sorbent assay (ELISA) of human TNF•

Materials

1) *1xPBS (pH 7.2, 150mM) and Tween 20:* 1xPBS was prepared by mixing 500mL (1.5M) of 10xPBS with 40g NaCl ( 1.36M, SRL Cat No. 1940103), 1 g KCl (26.83mM, SRL Cat No.1644133), 5.75g $Na_2HPO4$ (32mM, SRL No.1944143) and 1 g $KH_2PO4$ (14.69mM, SRL Cat No. 1649201). 1 litre of the prepared 1 x PBS was mixed with 0.5ml of Tween 20 (Polysorbate 20, SRL Cat No.1628154) to obtained the reagent.

2) *1xPBS (pH 7.2, 150mM) and 0.1% BSA:* 10mg of Bovine Serum Albumin (Bangalore Genei Catalog Number. 105256) was dissolved in 10ml of $1_XPBS$.

3) *Trizma Buffered Saline (TBS):* 485.6mg Trizma base (20mM, SRL Cat No. 2044122) was mixed with 1.752g NaCl (150mM, SRL Catalog Number.1940103). The pH of the mixture was adjusted to 7.3. After pH adjustment 200mg of 0.1 % BSA was added.

4) *5% skim milk in 1X PBS:* 5 gm of skimmed milk was dissolved in 100ml of 1xPBS.

5) *Primary antibody anti-human TNFa (R & D Systems Catalog Number.MAB 610) stock concentration:* A 500μg/ml stock solution of the primary antibody anti-human TNFa was prepared in 1xPBS (pH 7.2, 150mM) and 0.1% BSA.

6) Primary antibody anti-human TNFa (R & D Systems Catalog Number.MAB 610) working concentration: 4 μg/ml working concentration of the primary antibody anti-human TNFa was prepared by mixing 80μL of 500μg/ml in 10ml PBS.

7) *Biotinylated antibody working concentration(R & D Systems Catalog Number. BAF210):* 0.3 μg/ml of the biotinylated antibody working concentration was prepared by suspending 60μL of 50μg/mL biotinylated antibody stock in 10ml of trizma buffered saline (TBS).

8) *1x tetramethylbenzidine (TMB)/H2O2 substrate (Bangalore Genei Catalog Number.106035):* 20X stock solution of the tetramethylbenzidine (TMB)/$H_2O_2$ substrate was diluted with deionised water by a dilution factor of 1:20. The reagent is light sensitive and was kept in dark before and after use.

Method:

100μL (4 μg/ml) of primary antibody anti-human TNFa (R & D Systems Cat No.MAB 610) was added to each well of a 96 welled microtiter plate and stored overnight at 4°C to prepare a primary antibody anti-human TNFa coated microtiter plate.

Each well of the coated microtiter plate was washed four times with 300 μL PBS-Tween-20 (0.05%). The wells were then drained completely with a tissue paper until all the liquid is removed. 200 μL of 5% skimmed milk in 1xPBS was added to the well to block the plate and incubated at room temperature for 2 hours. 100 μL of either standard (R & D Systems Catalog Number. 210-TA., 1ng/ml, 0.5ng/ml, 0.25ng/ml, 0.125ng/ml, 0.062ng/ml, 0.031 ng/ml, 0.015ng/ml made in 1X PBS) or Control, Ex 1 or Ex 2 culture supernatant media obtained in Step 1 of Example 1 was added to the wells and incubated for 2 hours at room temperature. After the incubation period the well were washed four times with 300 μL of PBS-Tween-20 (0.05%). The wells were then drained completely with a tissue paper until all the liquid is removed. 100 μL (0.3μg/ml) of biotinylated antibody was added to each well and incubated for 2 hours at room temperature. Each well was then washed four times with 300 μL PBS-Tween-20 (0.05%). The wells were then drained completely with a tissue paper until all the liquid is removed. Each well was incubated with 1:200 dilutions in PBS of streptavidin-horseradish peroxidase (HRP) conjugates (Bangalore Genei Catalog Number HP 09) for 30 minutes. After the incubation period the wells were washed four times with 300 μL PBS-Tween-20 (0.05%). The wells were then drained completely with a tissue paper until all the liquid is removed. Each well was then incubated for 20 minutes with 100μL $1_X$ tetramethylbenzidine (TMB)/$H_2O_2$ substrate. The colour of the solution in each well was measured with a plate reader (from Thermo labsystems, iEMS Reader MF) and the readings were recorded. The reaction in each of the wells was stopped with 1 N $H_2SO_4$ and the OD at 450 nm was measured by the plate reader.

The readings were plotted on a graph and the slope of the graph was calculated. The amount of antigen/protein in the sample was estimated by the formula

Amount of antigen/protein in a sample (ng/mL) = (Absorbance of the sample at 450nm)

x 1/ A

where A is conversion factor, for conversion of optical density to absolute protein value in pictograms.

The amount of antigen in the supernatant media of control, Ex 1 and Ex 2 are provided in Table 2.

TABLE 2

| Examples | TNF • protein secretion (pg/mL) |
|---|---|
| Control (C) | 18.02 |
| Sample treated with 100μg of polymer of galacturonic acid per mL of media (Ex 1) | 26.73 |
| Sample treated with 500μg of polymer of galacturonic acid per mL of media (Ex 2) | 25.25 |

The data in Table 2 clearly shows that the keratinocyte cells treated with the polymer of galacturonic acid according to the present invention provides an increased secretion of TNF • protein as compared to the untreated keratinocyte cells (control).

## Claims

1. A topical or oral composition comprising:

    (i) 0.1 to 10 wt% of a polymer of galacturonic acid; and,
    (ii) a base;

    **characterised in that** the polymer of galacturonic acid has a molecular weight from 18 kDa to 35 kDa wherein the polymer of galacturonic acid is obtainable by a process comprising the steps of:

    (i) extracting *Camellia sinensis* in water at a temperature of 85 to 100°C;
    (ii) separating the extracted *Camellia sinensis* to obtain an aqueous extract;
    (iii) adding 30 to 70% ethanol-water solution to the aqueous extract to generate a precipitate;
    (iv) separating the precipitate of step (iii);
    (v) washing the precipitate of step (iv) with 70 to 95% ethanol-water solution;
    (vi) filtering and drying the washed precipitate of step (v) to obtain the extract of *Camellia sinensis* comprising a polymer of galacturonic acid with a molecular weight from 18 to 35kDa.
    (vii) separating the polymer of galacturonic acid with a molecular weight from 18 to 35kDa from said extract of *Camellia sinensis.*

2. A composition as claimed in Claim 1 wherein the polymer is a homopolymer of galacturonic acid.

3. A composition as claimed in Claim 1 or Claim 2 wherein the source of the polymer of galacturonic acid is *Camellia sinensis.*

4. A composition as claimed in Claim 3 wherein said *Camellia sinensis* is green tea leaves.

5. A composition as claimed in any one of the preceding claims wherein the topical composition is a leave-on or rinse-off product.

6. A composition as claimed in any one of the preceding claims wherein the topical composition is a skin composition in the form of a liquid, lotion, cream, foam, scrub, gel, soap bar, toner, a face mask, pad, patch, shampoos, conditioners, shower gels, antiperspirants, deodorants, depilatories, lipsticks, foundations, mascara, sunless tanners or sunscreen lotions.

7. A composition as claimed in claim 6 wherein the base is selected from water, liquid or solid emollients, emulsifiers, solvents, humectants, thickeners, powders or propellants.

8. A composition as claimed in claims 1 to 4 wherein the topical composition is a hair composition in the form of shampoos, conditioners, sprays, mousses, tonics, gels, oils, creams, air infused styling foams, rinses or lotions.

9. A composition as claimed in claim 8 wherein the base is selected from $C_1$-$C_6$ alcohols, lower alkyl acetate and mixtures thereof.

10. A composition as claimed in claims 1 to 4 wherein the composition is an oral composition in the form of a mouthwash, a toothpaste or a toothpowder.

11. An composition as claimed in claim 10 wherein the base is selected from water, silica or calcium carbonate.

12. Composition for use in a method of improving the immunity of skin, oral mucosa or scalp by applying to the desired surface a composition as claimed in any one of claims 1 to 11.

**Patentansprüche**

1.  Topische oder orale Zusammensetzung, umfassend:

    (i) 0,1 bis 10 Gew.-% eines Polymers der Galacturonsäure und
    (ii) ein Grundmaterial,

    **dadurch gekennzeichnet, dass** das Polymer der Galacturonsäure ein Molekulargewicht von 18 kDa bis 35 kDa aufweist, wobei das Polymer der Galacturonsäure durch ein Verfahren erhältlich ist, das die Schritte umfasst:

    (i) Extrahieren von *Camellia sinensis* in Wasser bei einer Temperatur von 85 bis 100°C,
    (ii) Abtrennen des extrahierten *Camellia sinensis,* um einen wässrigen Extrakt zu erhalten,
    (iii) Zugeben von 30 bis 70% Ethanol-Wasser-Lösung zu dem wässrigen Extrakt, um eine Ausfällung zu erzeugen,
    (iv) Abtrennen der Ausfällung des Schritts (iii),
    (v) Waschen der Ausfällung des Schritts (iv) mit 70 bis 95% Ethanol-Wasser-Lösung,
    (vi) Filtern und Trocknen der gewaschenen Ausfällung von Schritt (v), um den Extrakt von *Camellia sinensis* zu erhalten, umfassend ein Polymer der Galacturonsäure mit einem Molekulargewicht von 18 bis 35 kDa,
    (vii) Abtrennen des Polymers der Galacturonsäure mit einem Molekulargewicht von 18 bis 35 kDa von dem Extrakt der Camellia sinensis.

2.  Zusammensetzung, wie im Anspruch 1 beansprucht, wobei das Polymer ein Homopolymer der Galacturonsäure ist.

3.  Zusammensetzung, wie im Anspruch 1 oder Anspruch 2 beansprucht, wobei die Quelle des Polymers der Galacturonsäure *Camellia sinensis* ist.

4.  Zusammensetzung, wie im Anspruch 3 beansprucht, wobei die *Camellia sinensis* Blätter von grünem Tee darstellt.

5.  Zusammensetzung, wie in irgendeinem der vorhergehenden Ansprüche beansprucht, wobei die topische Zusammensetzung ein Leave-on- oder Abspülprodukt darstellt.

6.  Zusammensetzung, wie in irgendeinem der vorhergehenden Ansprüche beansprucht, wobei die topische Zusammensetzung eine Hautzusammensetzung in Form einer Flüssigkeit, Lotion, Creme, eines Schaums, Scheuermittels, Gels, Seifenstücks, Toners, einer Gesichtsmaske, eines Polsters, Tupfers, Shampoos, von Spülmitteln, Duschgelen, Antitranspirationsmitteln, Deodorants, Enthaarungsmitteln, Lippenstiften, Grundierungen, Mascara, Selbstbräunern oder Sonnenschutzlotionen ist.

7.  Zusammensetzung, wie im Anspruch 6 beansprucht, wobei das Grundmaterial unter Wasser, Flüssigkeit oder festen weichmachenden Mitteln, Emulgatoren, Lösungsmitteln, Feuchthaltemitteln, Verdickungsmitteln, Pulvern oder Treibmitteln ausgewählt ist.

8.  Zusammensetzung, wie in den Ansprüchen 1 bis 4 beansprucht, wobei die topische Zusammensetzung eine Haarzusammensetzung in Form von Shampoos, Konditioniermitteln, Sprays, Schäumern, Tonika, Gelen, Ölen, Cremes, Luftinfusionsstylingschäumen, Spülungen oder Lotionen vorliegt.

9.  Zusammensetzung, wie im Anspruch 8 beansprucht, wobei das Grundmaterial unter $C_1$-$C_6$-Alkoholen, Niederalkylacetat und Mischungen davon ausgewählt ist.

10. Zusammensetzung, wie in den Ansprüchen 1 bis 4 beansprucht, wobei die Zusammensetzung eine orale Zusammensetzung in Form eines Mundwassers, einer Zahnpasta oder eines Zahnpulvers ist.

11. Zusammensetzung, wie im Anspruch 10 beansprucht, wobei das Grundmaterial unter Wasser, Siliziumdioxid oder Calciumcarbonat ausgewählt ist.

12. Zusammensetzung zur Verwendung in einem Verfahren zur Verbesserung der Immunität von Haut, Mundschleimhaut oder Kopfhaut durch Aufbringen einer Zusammensetzung, wie in einem der Ansprüche 1 bis 11 beansprucht, auf die gewünschte Oberfläche.

**Revendications**

1. Composition topique ou orale comprenant :

   (i) de 0,1 à 10% en masse d'un polymère d'acide galacturonique ; et,
   (ii) une base ;

   **caractérisée en ce que** le polymère d'acide galacturonique présente une masse moléculaire de 18 kDa à 35 kDa où le polymère d'acide galacturonique peut être obtenu par un procédé comprenant les étapes de :

   (i) extraction de *Camellia sinensis* dans de l'eau à une température de 85 à 100°C ;
   (ii) séparation du *Camellia sinensis* extrait pour obtenir un extrait aqueux ;
   (iii) addition de solution d'éthanol 30 à 70 % - eau à l'extrait aqueux pour produire un précipité ;
   (iv) séparation du précipité de l'étape (iii) ;
   (v) lavage du précipité de l'étape (iv) avec une solution d'éthanol 70 à 95 % - eau ;
   (vi) filtration et séchage du précipité lavé de l'étape (v) pour obtenir l'extrait de *Camellia sinensis* comprenant un polymère d'acide galacturonique avec une masse moléculaire de 18 à 35 kDa
   (vii) séparation du polymère d'acide galacturonique avec une masse moléculaire de 18 à 35 kDa dudit extrait de *Camellia sinensis.*

2. Composition selon la revendication 1, dans laquelle le polymère est un homopolymère d'acide galacturonique.

3. Composition selon la revendication 1 ou la revendication 2, dans laquelle la source du polymère d'acide galacturonique est *Camellia sinensis.*

4. Composition selon la revendication 3, dans laquelle ledit *Camellia sinensis* est des feuilles de thé vert.

5. Composition selon l'une quelconque des revendications précédentes, dans laquelle la composition est un produit à laisser ou sans rinçage.

6. Composition selon l'une quelconque des revendications précédentes, dans laquelle la composition topique est une composition pour la peau dans la forme d'un liquide, d'une lotion, d'une crème, d'une mousse, d'un exfoliant, d'un gel, d'une barre de savon, d'un tonique, d'un masque pour le visage, d'un tampon, d'un patch, de shampoings, d'après-shampoings, de gels pour la douche, d'antiperspirants, de déodorants, de dépilatoires, de rouges à lèvres, de fonds de teint, de mascara, d'autobronzants ou de lotions d'écran solaire.

7. Composition selon la revendication 6, dans laquelle la base est choisie parmi l'eau, des émollients liquides ou solides, des émulsionnants, des solvants, des humectants, des épaississants, des poudres ou des agents de propulsion.

8. Composition selon les revendications 1 à 4, dans laquelle la composition topique est une composition pour les cheveux dans la forme de shampoings, d'après-shampoings, de pulvérisations, de mousses, de toniques, de gels, d'huiles, de crèmes, de mousses de coiffage imprégnées d'air, de rinçages ou de lotions.

9. Composition selon la revendication 8, dans laquelle la base est choisie parmi des alcools en $C_1$-$C_6$, un acétate d'alkyle inférieur et des mélanges de ceux-ci.

10. Composition selon la revendication 1 à 4, dans laquelle la composition est une composition orale dans la forme d'une eau de douche, d'un dentifrice ou d'une poudre dentaire.

11. Composition selon la revendication 10, dans laquelle la base est choisie parmi l'eau, la silice ou le carbonate de calcium.

12. Composition pour une utilisation dans un procédé d'amélioration de l'immunité de la peau, des muqueuses orales ou du cuir chevelu par application à la surface souhaitée d'une composition selon l'une quelconque des revendications 1 à 11.

**EP 2 931 376 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011069781 A1, Unilever
- US 2011097356 A
- EP 324720 A
- US 20070202233 A, Kato

### Non-patent literature cited in the description

- *Drug Discoveries & Therapeutics,* 2011, vol. 5 (1), 18-25
- Tea: Cultivation to Consumption. Chapman and Hall, 1992